# EUROPEAN PATENT SPECIFICATION

(11) **EP 1 884 214 B1**
(45) Date of publication and mention of the grant of the patent: **22.06.2011**
(21) Application number: 07013909.2
(22) Date of filing: 16.07.2007
(51) Int. Cl.: A61B 18/14

(54) **Electrosurgical treatment equipment**
Elektrochirurgische Behandlungsvorrichtung
Équipement de traitement électrochirurgical

(30) Priority: 31.07.2006 JP 2006207587
(43) Date of publication of application: 06.02.2008
(73) Proprietor: FUJIFILM Corporation, Tokyo (JP)
(72) Inventor: Oyatsu, Masayuki, Saitama-shi Saitama (JP)
(74) Representative: Klunker . Schmitt-Nilson . Hirsch

(56) References cited:
- EP-A- 1 728 462
- WO-A-99/00060
- US-A- 5 282 799
- US-A- 6 048 340
- US-A1- 2003 040 744
- US-A1- 2005 149 015

## Description

### Background of the Invention

### 1. Field of the Invention

The present invention relates to an electrosurgical treatment equipment for use in a procedure, such as incision, of a diseased mucous membrane by being passed through an treatment equipment-passage channel for an endoscope.

### 2. Description of the Related Art

Where a diseased point, such as a tumor, is found in a mucous membrane on a body cavity wall of the esophagus, stomach, duodenum, large intestine or the like, an electrosurgical treatment equipment is used to remove the diseased point of the mucous membrane. In the conventional procedure to remove such a diseased mucous membrane, endoscopic mucosal resection (EMR) has been conducted broadly with use of radio-frequency snare. In EMR, a significant diseased point could not be removed at one time thus requiring procedures over several times. Besides, there is a problem that the diseased point is possibly removed incompletely. For this reason, it becomes a recent practice to conduct endoscopic submucosal dissection (ESD) with use of an electrosurgical knife. In ESD, procedure is conducted in two stages, i.e. incising a mucous membrane at around a diseased point and then exfoliating the diseased mucous membrane including a part of its submucous layer from the muscular layer. The ESD scheme is advantageous in that a diseased point, even if significant, is to be removed completely of its mucous membrane by once procedure without encountering imperfect removal.

The electrosurgical treatment equipment, for use in ESD, is constructed to advance and retract its electrosurgical knife at a tip of a flexible sheath. Meanwhile, a handle section is coupled to a base of the flexible sheath so that the flexible sheath can be passed through an treatment equipment-passage channel for the endoscope. There is known, say, a disclosure in JP-A-2004-313537, as an electrosurgical treatment equipment of this type. In the known electrosurgical treatment equipment, an operating wire is passed through the interior of the flexible sheath wherein a knife as an electrosurgical tool section is coupled to a tip of the operating wire while the handle section is coupled to the base of the flexible sheath. The handle section has a slider. The slider is coupled with the base of the operating wire so that the knife can be advanced and retracted at the tip of the flexible sheath by pulling in and out the slider.

The knife is coupled with a circular or triangular platelike electrode at the tip of the rod electrode. It discloses also a structure that a hook knife is structured by bending the rod electrode at its tip. JP-A-2004-313537 describes that a procedure, such as incision and exfoliation, can be performed on a diseased mucous membrane by using an treatment equipment having a knife provided with a plate electrode. Furthermore, hemostasis is described also available by putting the plate electrode in the event that bleeding occurs in the course of a procedure.

U.S. Patent Application Publication No. 2005/0149015 A1 discloses an electro-cautery catheter which includes a distal electrode tip portion that acts as a hemostat and a cutting wire for making incisions in tissue. The cutting wire can be displaced between extended and retracted positions and is electrically isolated from the electrode tip. The electrode tip may be disposed spirally on an outer peripheral surface of adistal end of the catheter in order to provide hemostatic therapy to selected tissue.

In the usual ESD, when a diseased mucous membrane is incised/exfoliated by use of an electrosurgical treatment equipment, marking is previously made on a region to incise, in order to prevent the imperfect removal of a diseased tissue and to suppress the damage to the health tissue to the minimal extent. Meanwhile, in order to swell the mucous membrane point to incise, a tissue-expander liquid such as of physiological saline and hyaluronic acid is locally injected to the submucous layer. This is followed by incision of the mucous membrane wherein the electrosurgical knife is used in such incision. When exfoliating the mucous membrane, the electrosurgical knife is also used. The submucous layer, in its relevant point, is exfoliated by inserting the electrosurgical knife therein.

In the procedure, caution must be paid not to invade the muscular layer existing in the underneath of the submucous layer. Namely, in case the muscular layer be touched by the electrosurgical procedure section made by an electrosurgical knife, etc., the muscular layer is possibly perforated to cause a quantity of bleeding. Accordingly, when conducting a ESD procedure, careful handling is needed so as not to put the electrosurgical procedure section in contact with the muscular layer. This requires the high degree of experience and skill.

Particularly, the shape is important for the electrosurgical knife for use in incising a mucous membrane. Meanwhile, the electrosurgical knife is required limited in its projection out of the flexible sheath to a degree not reaching the muscular layer. Meanwhile, exfoliating a mucous membrane, following incision, is desirably by inserting the electrosurgical knife in the submucous layer and moving it in a manner swinging left and right. Consequently, the electrosurgical knife suited in incising a mucous membrane is not necessarily beneficial in exfoliating the membrane. It is not proper to exfoliate a mucous membrane by means of the one for incision use. Accordingly, there is a need of such a procedure using two types of electrosurgical treatment equipments, i.e. incision is performed by means of an incising electrosurgical treatment equipment passed through an endoscopic treatment equipment-passage channel, followed by inserting an exfoliating electrosurgical treatment equipment into the treatment equipment-passage channel in an exchanging fashion. This however is troublesome in handling thus forming a factor to increase the time of procedure.

As was already explained, JP-A-2004-313537 describes that two types of procedures, i.e. mucous membrane incision and exfoliation, are to be performed by use of an electrosurgical treatment equipment having a plate-electrode knife. It can be considered not impossible to perform a series of procedures by use of such electrosurgical treatment equipments. Nevertheless, the electrosurgical knife is considered not proper for the procedures even if using a circular or triangular electrode or a hook knife bent at the tip of its rod electrode. In any of the procedures, there still remains a fear of bleeding, perforation, etc. Therefore, there is an essential need to use electrosurgical treatment equipments suited for the respective procedures of incision and exfoliation of a mucous membrane in respect of safety and positiveness even where taking account of troublesomeness in handling.

### Summary of the Invention

The present invention, made in view of the foregoing point, aims at providing an electrosurgical treatment equipment capable of performing two types of procedures continuously by incorporating respective procedure section suited for the two procedures, e.g. incision and exfoliation, without the need of exchangeably inserting those in the endoscope.

In order to solve the foregoing object, the present invention is an electrosurgical treatment equipment as recited in claim 1.

The electrosurgical treatment equipment is provided with two types of an electrosurgical procedure section at the tip of the flexible sheath. With each of the electrosurgical procedure section, an RF current is selectively supplied to a subject. One of the two types of procedure sections is an electrosurgical knife. The electrosurgical knife is suited to cauterize and incise a body tissue. The other of procedure sections is an RF current conductor provided in a side surface at a tip of the flexible sheath. When the RF current conductor is connected with the RF power source, the opposite point is cauterized. Accordingly, the electrosurgical treatment equipment of the invention is suited for removing a diseased mucous membrane according to an ESD scheme. However, the electrosurgical treatment equipment is not exclusive for such a procedure. In brief, it is capable of performing a procedure with the electrosurgical knife projected at the flexible sheath in the state passed through an endoscopic treatment equipment-passage channel, and a procedure to perform in a manner swinging the flexible sheath at its tip.

The electrosurgical knife is handled in a manner to cut a body tissue. Accordingly, it is desired to restrict the projection out of the flexible sheath in viewpoint of safety. For this reason, the handle section can be structured to comprise a main stem coupled to the base of the flexible sheath and a slider slidably fit over the main stem axially of the main stem and coupled with another end of the cord. The electrosurgical knife is restricted in its greatest projection out of a tip of the flexible sheath by the provision of a projection restricting section.

Desirable id the form of the electrosurgical knife that is to advance and retract at the tip of the flexible sheath. Namely, it includes suitable forms, e.g. a rod electrode, a hook knife form and an IT knife fit with an electric insulation member at its tip. In any case, the electrosurgical knife is restricted in its projection out of the flexible sheath by means of the projection restricting section. Where used in incising a mucous membrane, the projection out of the flexible sheath is established equal to or greater than the thickness of the mucous layer and equal to or smaller than the totalized dimension in thickness of a mucous layer and a submucous layer. This allows for incision of a mucous layer. In handling, there is no occurrence of a situation that the electrosurgical knife touches a muscular layer through the submucous layer, and hence no occurrence of bleeding, perforation or the like.

Meanwhile, the RF current conductor, formed in the side surface at the tip of the flexible sheath, is quite convenient in exfoliating a mucous membrane. Exfoliating a mucous membrane can be by connecting a procedure section having an RF current conductor to the RF power source and inserting it in a submucous layer and moved in a manner of being swung left and right. In order for such handling, the RF current conductor is given a width comparatively narrow in respect of current density. Meanwhile, the flexible sheath is desirable increased in its axial length to a certain extent. Meanwhile, by providing the RF current conductor in two positions having a 180-degree angular relationship in the outer peripheral surface of the flexible sheath, mucous-membrane exfoliation can be effected by swing-moving the flexible sheath.

The electrosurgical knife and the RF current conductor can be connected respectively to RF power sources independent mutually. However, because there is no possibility to simultaneously supply RF currents to the electrosurgical knife and the RF current conductor when performing procedures of incision and exfoliation of a mucous membrane, it is desirable to provide those to be electrically connected to a single RF power source. Connection can be provided for switching between the RF power source and the electrosurgical knife or RF current conductor through a switch section. In this case, the electrosurgical knife when not operated is retracted in a flexible sheath formed by an electrical insulation member. Namely, when supplying an RF current to the RF current conductor, the electrosurgical knife is retracted in the flexible sheath and kept in a state not contacted with a body cavity wall. Consequently, the electrosurgical knife may be held connectable with an RF power source so that the RF current conductor can be first connected with the RF power source when exfoliating a mucous membrane is made ready to perform by supplying an RF current to the RF current conductor in the state the electrosurgical knife is retracted in the flexible sheath. In such a case, the electrosurgical knife is desirably electrically shut off from the RF power source. However, where the flexible sheath internally is of a liquid-tight structure for example, those may be in a connected state at all times without encountering any hindrance.

### Brief Description of the Drawings

Fig. 1 is an overall construction view of an electrosurgical treatment equipment showing one embodiment according to the present invention;
Fig. 2 is an exterior view of the Fig. 1 electrosurgical treatment equipment at its tip;
Fig. 3 is a sectional view of the Fig. 1 electrosurgical treatment equipment at the tip of the Fig. 1 electrosurgical treatment equipment;
Fig. 4 is a sectional view in the state an electrosurgical knife is retracted in a flexible sheath, in a position as viewed 90-degree different from that in Fig. 3;
Fig. 5 is a structural explanatory view of the tip of the flexible sheath in the state the electrosurgical knife is projected;
Fig. 6 is a sectional view of the Fig. 1 electrosurgical treatment equipment, taken in a direction of a main stem and along the slit groove thereof;
Fig. 7 is a sectional view taken on line X-X in Fig. 6;
Fig. 8 is an exterior view of an electrosurgical treatment equipment showing one embodiment of the invention, in the state passed through an treatment equipment-passage channel for an endoscope ;

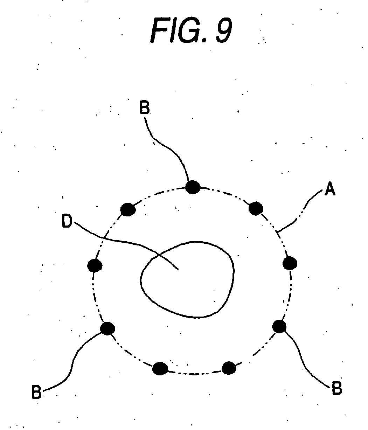

Fig. 9 is a plan view showing the state marks are put on a diseased mucous membrane;
Fig. 10 is a sectional view of a tissue showing the state that local injection is being made to the diseased point of the mucous membrane;

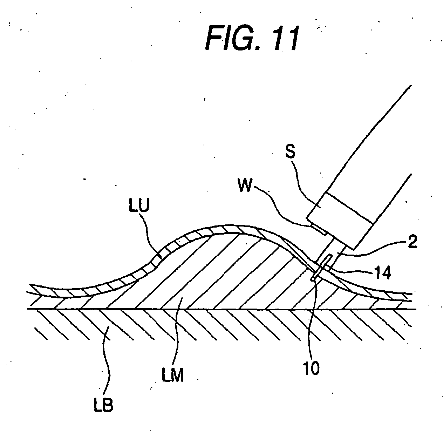

Fig. 11 is a sectional view of the tissue showing the state that incision is being performed by use of the electrosurgical treatment equipment;
Fig. 12 is a plan view including the diseased point of the mucous membrane showing the state that incision with the electrosurgical treatment equipment is completed;
Fig. 13 is a sectional view of the tissue showing the state that exfoliation is made of the mucous membrane;
Fig. 14 is a sectional view showing a second embodiment of the invention, taken in a direction along the slit groove of the main stem in an incisive position;
Fig. 15 is a sectional view showing the second embodiment of the invention, taken in the direction along the slit groove of the main stem in an exfoliative position;
Fig. 16 is a structural explanatory view showing a third embodiment of the invention, illustrating a coupling of between a terminal pin and a swing arm; and
Fig. 17 is an explanatory view showing the third embodiment, illustrating a connection state of a terminal pin, a conductor wire and a wiring cable.

### Detailed Description of the Invention

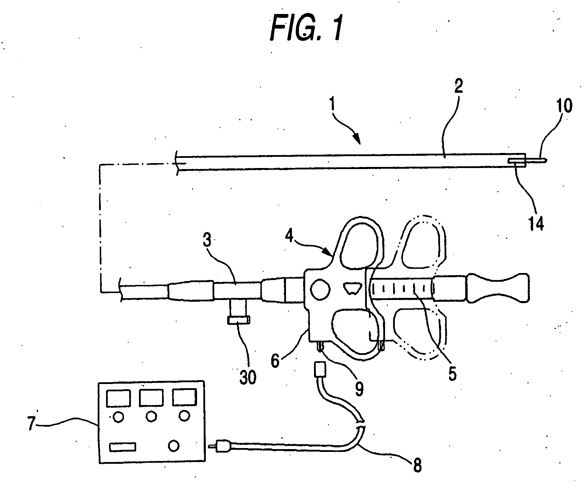

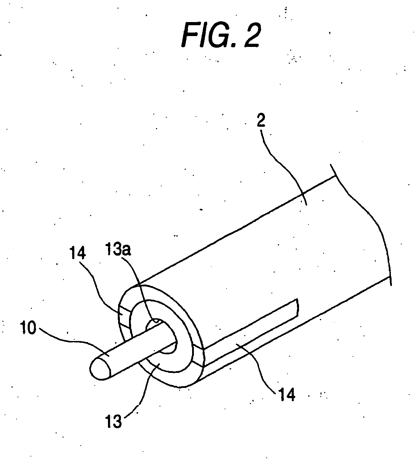

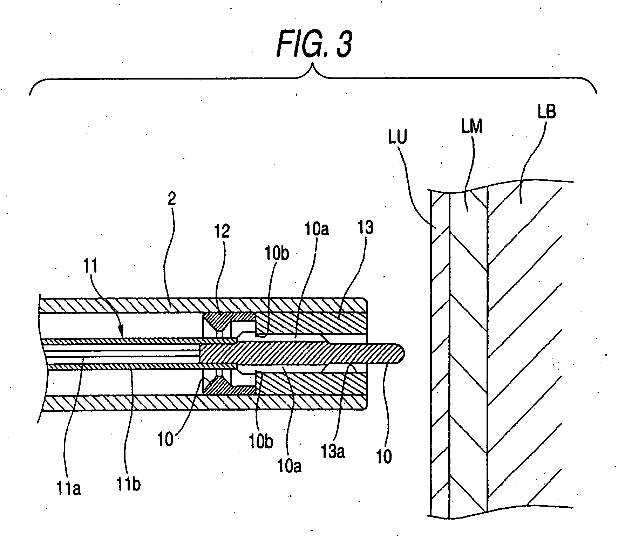

Based on the drawings, explanation will now be made on embodiments according to the present invention. The embodiments explain on the mechanism adapted to incise and exfoliate a diseased mucous membrane. Note that the electrosurgical treatment equipment in the invention is naturally to be applied to other types of procedures. Fig. 1 shows an overall construction of an electrosurgical treatment equipment, Fig. 2 an exterior view of a tip thereof, and Fig. 3 a sectional view taken on line X-X in Fig. 2.

In Fig. 1, reference numeral 1 designates an electrosurgical treatment equipment. The electrosurgical treatment equipment 1 has a flexible sheath 2 made up by an elongate insulation tube. The flexible sheath 2 has a base that is connected with a connection pipe 3 while the connection pipe 3 is coupled with a handle section 4 at the end thereof. The handle section 4 is structured with a main stem 5 that is coupled to the connection pipe 3 and a slider 6 that is fit axially movably over the main stem 5 and to be handled by operator's fingers.

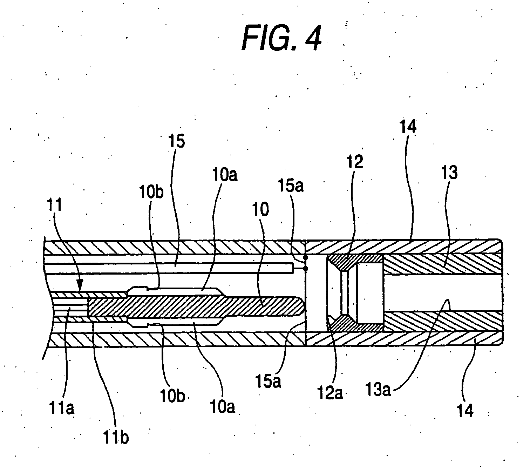

At the tip of the flexible sheath 2, an electrosurgical knife 10 is arranged formed of an electrode member in a circular rod form having a semispherical tip, as shown in Fig. 2. As shown in Fig. 3, the electrosurgical knife 10 is coupled with a flexible cord 11. The flexible cord 11 is formed by a conductive wire 11a covered with an insulation coat 11b. The flexible cord 11 extends through the interior of the flexible sheath 2 and connection pipe 3, the base of which is coupled to the slider 6 of the handle section 4. Accordingly, by moving the slider 6 forward over the main stem 5 as shown in the solid lines in Fig. 1, the electrosurgical knife 10 is positioned projecting at the tip of the flexible sheath 2 as shown in Fig. 3. In this position, the electrosurgical knife 10 is operative, i.e. in an incisive position. In case the slider 6 is moved rearward from the incisive position into a position shown with the virtual lines in the figure, the electrosurgical knife 10 is completely retracted in the flexible sheath 2 as shown in Fig. 4. In this position, exfoliation is possible to perform on a mucous membrane under the supply of a radio-frequency current to RF current conductors 14, referred later.

At the tip of the flexible sheath 2, provided are an introduction ring 12 and an insulation tip 13 as shown in Fig. 3. The insulation tip 13 is a pipe-like member formed of an electric insulation material having heat resistance and rigidity, e.g. ceramic. The introduction ring 12 is not necessarily formed of an electric insulation material but requires heat resistance. The introduction ring 12 and the insulation tip 13 are received in the tip of the flexible sheath 2 and fixed therein through the means of an adhesive or the like.

The introduction ring 12 has an introducing surface 12a formed slant at a predetermined angle relative to the outer periphery thereof while the insulation tip 13 has a reception passage 13a that receives the electrosurgical knife 10 therein. Accordingly, the electrosurgical knife 10 is axially aligned by being guided by the introduction ring 12 at its introducing surface 12a so that it can project to the outside through the reception passage 13a of the insulation tip 13. The electrosurgical knife 10 is received in a play-fit manner in the reception passage 13a of the insulation tip 13. As shown in Figs. 3 and 4, a plurality of vanes 10a are formed in an outer peripheral surface of the electrosurgical knife 10 so that the electrosurgical knife 10 can be positioned stable without positional deviations at its axis when passed in the insulation tip 13. The vanes 10a are each formed with a stopper wall 10b at the base thereof in order to restrict the projection amount of the electrosurgical knife 10 out of the flexible sheath 2, so that those at the stopper walls 10b can abut against the base-end face of the insulation tip 13. Accordingly, this constitutes a greatest-projection regulating section that regulates the greatest projection of the electrosurgical knife 10 out of the flexible sheath 2. Here, the electrosurgical knife 10, when projected greatest at the tip of the flexible sheath 2, has a projection that is restricted in amount equal to or greater than a thickness of the mucous layer LU and equal to or smaller than the total thickness of the mucous layer LU and submucous layer LM. Accordingly, the electrosurgical knife 10 is not to reach a muscular layer LB even if it is projected greatest in the state the flexible sheath 2 is put at its tip surface in contact with the mucous layer LU.

As apparent in Fig. 2, the flexible sheath 2 is arranged with electrode members at the tip thereof, in both left and right positions, i.e. in positions having a 180-degree angular relationship. Those electrode members constitute RF current conductors 14 that are to be connected to an RF power source. The RF current conductors 14 are provided exposed in the outer peripheral surface of the flexible sheath 2, each of which has a width comparatively small and an axial length slightly greater than the fit length of the insulation tip 13.

The RF current conductors 14 are respectively connected with wires 15a, as shown in Fig. 4. The wires 15a extend along the inner surface of the flexible sheath 2 and united into one as a wire cable 15, thus extending from the flexible sheath 2 to an interior of the main stem 5 of the handle section 4 through the connection pipe 3.

The handle section 4 is removably connected with a cable 8 of from the RF power source 7 so that RF power can be fed to the electrosurgical knife 10 and RF current conductors 14. Meanwhile, the subject is placed in contact with not-shown opposite pole plates, which causes cauterization at the contact between the electrosurgical knife 10 and RF current conductors 14 and the body-cavity wall. The slider 6 has a terminal pin 9, allowing the cable 8 to be connected to the terminal pin 9.

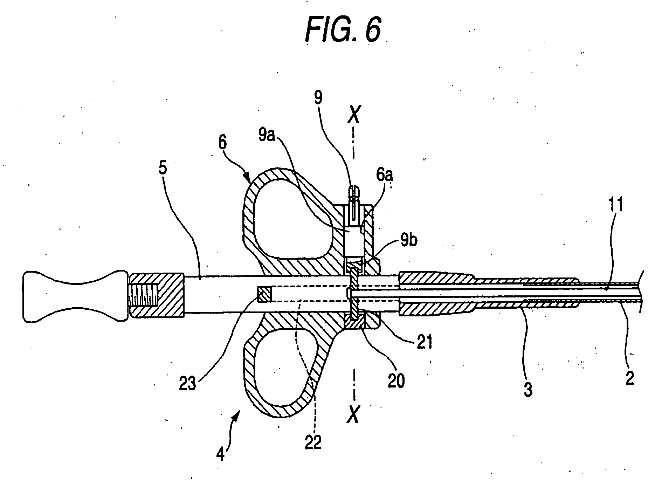

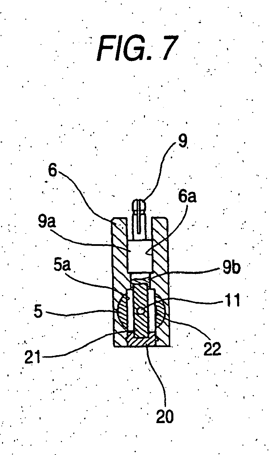

Figs. 6 and 7 show a structure of the handle section 4. As apparent from the figures, a slit groove 5a is formed in a manner penetrating in a left-right direction, in the main stem 5 over which the slider 6 is fit. The flexible cord 11, connected to the electrosurgical knife 10, extends to the slit groove 5a of the main stem 5 through the connection pipe 3. The slider 6 is removably attached with a coupling block 20. The coupling block 20 is coupled with an electrode member 21 formed of a conductive material and for pulling the flexible cord 11 in and out. Thus, the flexible cord 11 is coupled to the electrode member 21 while the conductor wire 11a of the flexible cord 11 is electrically connected with the electrode member 21.

The connection pin 9 has a rod portion 9a that is fit in an attachment hole 6a provided in the slider 6. The rod portion 9a is extended with an electric connection 9b on the side opposite to the connection pin 9. The electric connection 9b is received with a tip of the electrode member 21 that is coupled to the coupling block 20 removably attached in the slider 6. This places the conductor wire 11a of the flexible cord 11 in electrical connection with the connection pin 9, to supply RF current to the electrosurgical knife 10. Incidentally, the slider 6 and the coupling block 20 are formed of an electrically insulating material, e.g. synthetic resin.

Meanwhile, the wire cables 14 of from the RF current conductors 14 are connected to the a wiring pattern 22 formed on the surface of the slit groove 5a. The wiring pattern 22 is covered with an insulation coat. A contact 23 is provided in a position where the electrode member 21 lies when the slider 6 is moved to the position shown by the virtual lines in Fig. 1, i.e. moved to the exfoliative position. The contact 23 is in electrical connected with the wiring pattern 22. Accordingly, when the slider is moved to the exfoliative position, the electrode member 21 is placed in connection with the contact 23, to supply power to the RF current conductors 14.

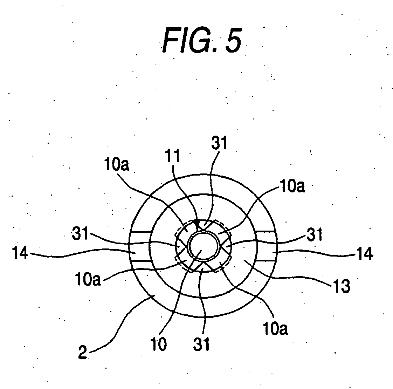

Furthermore, the electrosurgical treatment equipment 1 has a fluid supply/drain passage. The fluid supply/drain passage is structured with an interior path of the flexible sheath 2. A fluid connector 30 is formed on the connection pipe 3 coupled with the flexible sheath 2, to connect with a syringe, tube or the like. Meanwhile, a plurality of passages 31 are formed between adjacent ones of the vanes 10a, 10a of the electrosurgical knife 10, at the tip of the flexible sheath 2 (see Fig. 5). Accordingly, by connecting a liquid pressure-feed section to the fluid connector 30, a tissue-expander liquid, such as physiological saline or hyaluronic acid, is supplemented to a point to swell. Meanwhile, by connecting a vacuum section, aspiration is made available from the body interior.

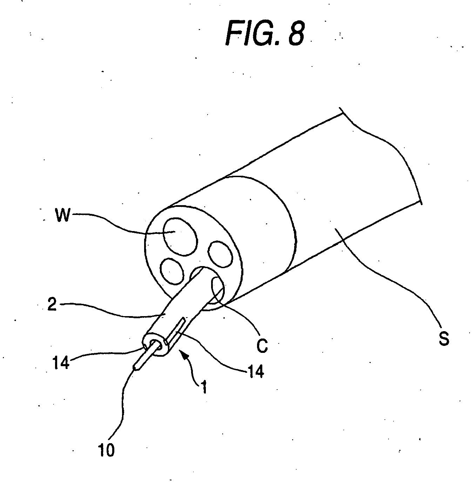

The electrosurgical treatment'equipment 1 thus constructed is to be inserted in a body cavity through an treatment equipment-passage channel C provided in an endoscope insertion tube S having an observation portion W, as shown in Fig. 8. When there is a diseased mucous membrane in a body cavity wall, say, of the esophagus, stomach, duodenum or large intestine, it is used to exfoliate and remove the diseased mucous membrane. Therefore, explanation is made on an example of the procedure to remove a diseased mucous membrane. The procedure is conducted when a diseased point is confirmed existing in a mucous membrane as a result of examination with an endoscope S.

At first, marking is made on a mucous membrane where there exists a diseased region D to remove, in a manner to surround the diseased region D. The marking is limited in area that can remove the diseased region can be removed completely with less damage to the healthy mucous membrane to a possible extent. Marking can be made, for example, by putting cauterization spots B at required points in a peripheral region A of the diseased mucous membrane D. The electrosurgical treatment equipment 1 can be used in forming such cauterization spots B. Namely, the endoscope insertion tube S at its tip is put facing to the diseased mucous membrane D at its outer edge with a predetermined spacing. In this state, the electrosurgical treatment equipment 1 is inserted in the treatment equipment-passage channel C and placed at its tip in contact with a surface of the mucous membrane. At this time, the electrosurgical knife 10 is in a state retracted to a position closer to the base end in the rigid cylinder 2, as shown in Fig. 4. Here, the flexible sheath 2 of the electrosurgical treatment equipment 1 has a tip made as an annular end wall having a dimension totalized over the thickness of the flexible tube 2 and the thickness of the insulation tip 12. The annular end wall is to contact with a mucous membrane surface at a broad area.

In this state, the handle section 4 of the electrosurgical treatment equipment 1 is operated to move the slider 6 forward to the solid-lined position in Fig. 1. Due to this, the electrosurgical knife 10 projects out of the flexible sheath 2. In this state, in case an RF current is applied to the electrosurgical knife 10, the mucous membrane is cauterized at a point the electrosurgical knife 10 is in contact therewith, thus effecting a marking. Here, in making such a marking, the electrosurgical knife 10 is not required to penetrate through a mucous layer LU. It is satisfactory to cause a cauterization in the mucous membrane surface to an extent that recognition is possible from an image obtained through the observation portion W. Because the electrosurgical knife 10 is always in electrical connection to the connection pin 9 through the conductor wire 11a of the flexible cord 11 and the electrode member 21, current supply is available even in a state the electrosurgical knife 10 is projected slightly out of the flexible sheath 2 provided that the cable 8 of from the RF power source 7 is connected to the connection pin 9 of the slider 6. Here, because the greatest projection of the electrosurgical knife 10 out of the flexible sheath 2 is given smaller than the total thickness of the mucous layer LU and submucous layer LM, even in case the slider 6 is moved fully in its stroke, the electrosurgical knife 10 is not to reach a contact point with a muscular layer LB. Thus, there is no fear to invade the muscular layer LB. Note that, marking can be by use of other types of treatment equipments. Meanwhile, provided that the mucous-membrane region to remove is to be recognized through the observation portion W, there is no need to employ the technique of cauterization as in the foregoing.

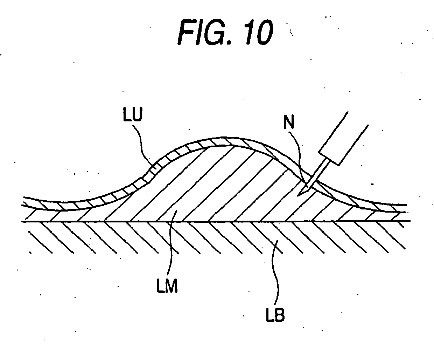

Then, tissue-expander liquid, such as.hyaluronic acid, is locally injected to the diseased mucous membrane D, specifically to the submucous ,layer LM, as shown in Fig. 10. In order for this, the electrosurgical treatment equipment 1 is once withdrawn out of the treatment equipment-passage channel. In place therewith, a local-injection section; having an injection needle N at the tip of the flexible tube, is inserted in the treatment equipment-passage channel C. Here, the injection needle N is to be punctured to the submucous layer LM through the mucous layer LU, to inject a tissue-expander liquid. As a result, the submucous layer LM swells' and rises. In this manner, the submucous layer LM is swelled in order to conduct the procedure smoothly and safely through fully isolating the mucous layer LU from the muscular layer LM.

After swelling the submucous layer LM, the local injection section is withdrawn out of the treatment equipment-passage channel C. The electrosurgical treatment equipment 1 is again inserted in the treatment equipment-passage channel C, to project the flexible sheath 2 by a predetermined amount. In this case, the cable 8 of from the RF power source 7 is kept connected to the connection pin 9. However, because the supply of power from the RF power source 7 is controlled by a switch section (not shown) separately provided, power is not necessarily immediately supplied in this state.

The slider 6 is moved forward from the virtual-lined position in Fig. 1 to the solid-lined position, by manual operation or so. Due to this, the electrosurgical knife 10 is projected from the tip of the insulation tip 13. However, once the vanes 10a at the stopper walls 10b come into abutment against the base-end face of the insulation tip 13, the electrosurgical knife 10 no longer projects out of the flexible sheath 2 furthermore. In this state, while supplying a current from the RF power source 7 to the electrosurgical knife 10 through the conductor wire 11a of the flexible cord 11, the electrosurgical treatment equipment 1 is operated in a manner to push the entire thereof out of the treatment equipment-passage channel C. Due to this, the mucous layer LU is cut by the electrosurgical knife 10 wherein the flexible sheath 2 at its tip face, specifically the flexible sheath 2 and insulation tip 13 at its tip face, is pushed upon the body cavity wall. The supply of power from the RF power source 7 is controlled by means of a switch section (not shown) provided separately. As a result, starting a cauterization by means of the electrosurgical knife 10, the electrosurgical knife 10 is lead down to the submucous layer LM through the membrane layer LU, thus incising the diseased mucous membrane D.

In this state, by moving the endoscope insertion tube S or bending its bend portion under the observation through the viewing window W, incision is proceeded with a movement along the cauterization spots B. Because the electrosurgical knife 10 is restricted in its projection amount and the submucous layer LM is swelled by local injection, the mucous layer LU can be positively incised unless the mucous membrane at its surface is deformed extremely by the tip of the flexible sheath 2. Moreover, the mucous layer LU can be incised without giving any damage to the muscular layer LB. Here, because the electrosurgical knife 10 is restricted in its projection amount out of the flexible sheath 2, the muscular layer LB is not invaded when the slider 6 is'placed in an incisive position advanced fully in stroke, thus assuring safe operation without incurring such a situation as perforation or bleeding.

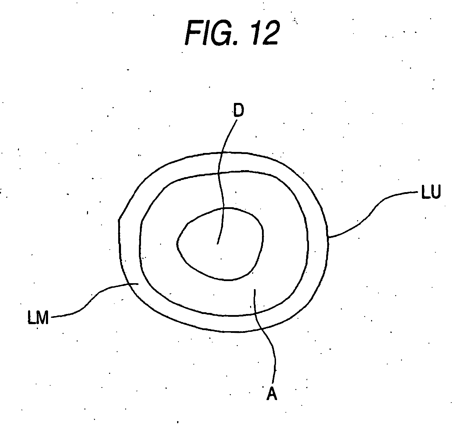

Incision is made throughout the periphery of the marked region as shown in Fig. 12, with a result that the mucous layer LU is incised at around the periphery of the diseased mucous membrane D, thus resulting in a state the submucous layer LM is exposed. Incidentally, the diseased mucous membrane D in its entirety was incised at one time in Fig. 12. Where such a diseased mucous membrane D is significantly broad, incision may be in a part followed by exfoliation, referred later, so that the procedure can be repeated a plurality of number of times.

The mucous layer LM cannot be removed by merely incising the diseased mucous membrane D at the entire periphery thereof. Namely, because the mucous layer LU and the muscular layer LB are connected through a fibrous submucous layer LM, there is a need to exfoliate the mucous layer LU by cutting the fibrous tissue. It is not desirable to exfoliate such a mucous membrane by use of an electrosurgical knife 10 in an elongate rod form and restricted in projection amount, in respect of efficiency, safety in handling and so on. Therefore, in place of the electrosurgical knife 10, exfoliation procedure is performed by means of the RF current conductors 14 provided in the flexible sheath 2 at the tip outer periphery thereof.

In order for this, the slider 6 constituting the handle section 4 is operated in a manner being slid rearward over the main stem 5. This pulls the flexible cord 11, coupled to the slider 6, toward the base end. The electrosurgical knife 10 is retracted in the flexible sheath 11. When the slider 6 is moved to the stroke end, i.e. to the virtual-lined position in Fig. 1, the electrode member 21, connected to the coupling block 20 and electrically connected to the terminal pin 9, is placed in abutment against the contact 23, thus resulting in a state that power is to be supplied to the RF current conductors 14 through the wiring pattern 22 and wiring cable 15. At this time, power is desirably shut down from being supplied to the electrosurgical knife 10. By separately providing a switch section, the supply of power to the electrosurgical knife 10 can be shut down when power is being supplied to the RF current conductors 14. By observing the tip of the flexible sheath 11 through the observation portion W of the endoscope S, confirmation is made on the position of RF current conductors 14. The RF current conductors 14 are desirably regulated nearly parallel with the mucous layer LU. Where there is a need for directional regulation as a result of observation through the observation portion W, directional adjustment is made in a manner twisting the flexible sheath 2.

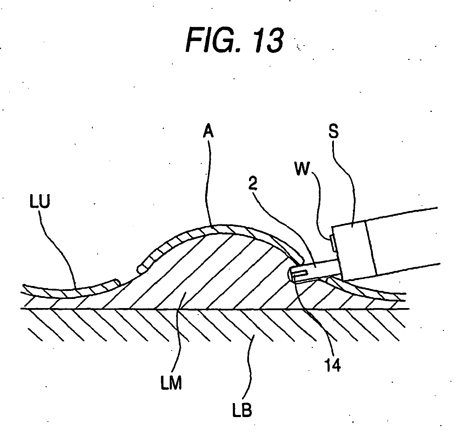

In this state, the flexible sheath 2 is moved in the exposed portion of the submucous layer LM caused due to the incision by means of the electrosurgical knife 10, as shown in Fig. 13. While supplying an RF current from the RF power source 7, the flexible sheath 2 is moved horizontally or swung, thereby cutting the submucous layer LM in a manner cauterizing it through the action of the RF current. The movement can be easily done by incurvating the tip of the endoscope insertion tube S or so. As a result, the mucous membrane can be exfoliated with swiftness and efficiency. Here, because the RF current conductors 14 are adjusted nearly parallel with the mucous layer LU, exfoliation can be effected with smoothness and swiftness. Moreover, because the RF current conductors 14 are nearly parallel also with the muscular layer LB, there is no fear that the RF current conductors 14 be put in contact with the muscular layer LB in the procedure.

Here, even in the exfoliative position, the electrosurgical knife 10 is in connection to the RF power source 7. However, because the electrosurgical knife 10 is retracted in the electrically-insulating flexible sheath 2 and positively isolated from the body cavity wall, there is no possibility that current flow from the electrosurgical knife 10 to the opposite pole plate. The incised mucous layer LU can be exfoliated by the operation of the RF current conductors 14 only.

Incidentally, where a procedure is performed based on an RF current of from the RF power source 7 during incision or exfoliation, there is a possibility to cause bleeding at the procedure site, etc. In such a case, the bleeding point can be irrigated rapidly by supplying a cleaning liquid under pressure into the flexible sheath 2 through the connection port 3a of the connection pipe 3. The cleaning liquid can be supplied through the reception passage 13a of the insulation tip 13 where the electrosurgical knife 10 is retracted in the flexible sheath 2, or through the plurality of passages 31 given between the vanes 10a, 10a of the electrosurgical knife 10. Meanwhile, a tissue-expander liquid is necessarily supplied when exfoliating a mucous membrane. There is a possible case that a swelled region shrinks due to the outflow in the course of procedure or the imbibition in the body despite a tissue-expander liquid is already poured locally. In order to avoid this, mucous-membrane incision or exfoliation can be made while supplying a tissue-expander liquid in order to keep the mucous submucous layer LM in a swelled state.

As described so far, two types of procedures, i.e. mucous membrane incision and exfoliation, can be.performed by use of one single electrosurgical treatment equipment 1. Moreover, incision is by use of the electrosurgical knife 10 made by a thin rod-like electrode whereas exfoliation is by means of the RF current conductors 14 provided a pair in side surfaces of the flexible sheath 2 and having a predetermined width and length. By making the electrosurgical knife 10 and RF current conductors 14 in a structure suited for the purpose, a diseased mucous membrane D can be removed smoothly by continuous procedure without requiring a troublesome handling, e.g. exchanging the treatment equipment at the treatment equipment-passage channel C. Moreover, the diseased mucous membrane D can be removed completely while reducing the damage to the health tissue to the minimum. Furthermore, because the electrosurgical knife 10 is regulated in projection amount out of the flexible sheath 2, procedure can be done with safety but less in invasion thus assuring safe operation without incurring such a situation as perforation or bleeding in the procedure.

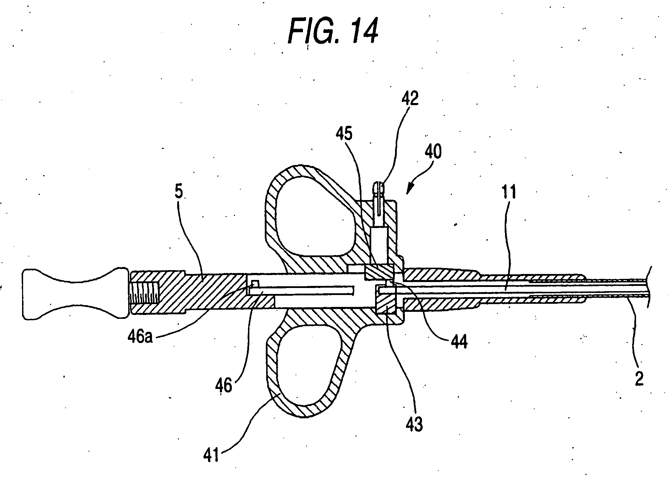

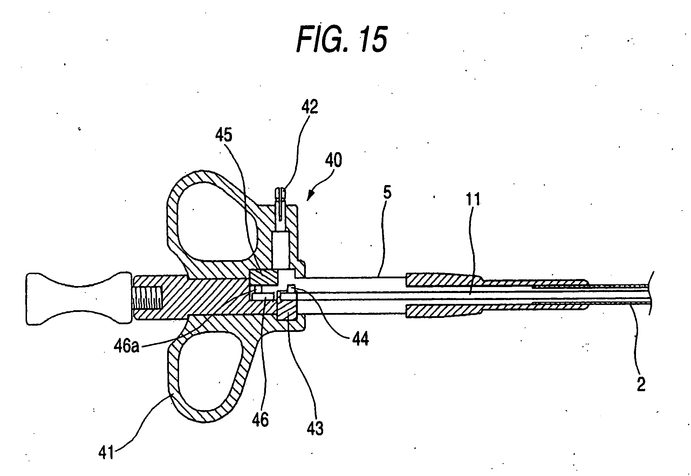

Figs. 14 and 15 shows a second embodiment according to the invention. In this embodiment, a slider 41 constituting a handle section 40 is arranged with a terminal pin 42 for connection to an RF power source wherein the terminal pin 42 is to be connected, by switchover, to an electrosurgical knife 10 and RF current conductors 14. For this reason, the slider 41 is coupled therein with a clamp member 43 formed of an electrical insulation material wherein the clamp member 43 is coupled with a flexible cord 11 at its end. The flexible cord 11 has a conductor wire 11a electrically connected to a contact 44 provided in the clamp member 43. Furthermore, the slider 41 is also provided with a slide electrode 45 to be electrically connected to the terminal pin 42. The slide electrode 45 is to be slid axially of the main stem 5 by means of a not-shown knob. The slide electrode 45, if slid to any position, is to be in electrical conduction to the terminal pin 42. Furthermore, the wiring cable 15 of from the RF current conductors 14 is connected to a contact member 46 provided fixedly on a predetermined position of the main stem 5. The contact member 46 has a contact 46a.

With the above structure, the electrosurgical treatment equipment 1 is put in an operative position, i.e. in a position the slider 41 is positioned forward, as shown in Fig. 14, to project the electrosurgical knife 10 out of the tip of the flexible sheath 2. By placing the slide electrode in a forward position, the contact 44 electrically connected with the conductor wire 11a of the flexible cord 11 is brought in a state connected with the slide electrode 45 and terminal pin 42. Accordingly, incision is made possible to perform by feeding a current to the electrosurgical knife 10.

The slider 41, if slid rearward, takes the position shown in Fig. 15, thus placing the electrosurgical tool 1 in an exfoliative position. Namely, because the clamp member 43 coupled to the slider 41 moves, the flexible cord 11 coupled to the clamp member 43 is pulled out to retract the electrosurgical knife 10 coupled to the tip of the flexible cord 11 in the flexible sheath 2. In this state, in case the slide electrode 45 is moved backward, the slide electrode 45 is placed out of conduction from the contact 44 that is closer to the flexible cord 11, thus being placed into electrical connection with the contact 46a of the contact member 46 connected to the wiring cable 46 and hence conducted to the RF current conductors 14. As a result, exfoliation can be performed on the mucous membrane continuously from incision, without exchanging the treatment equipment in the treatment equipment-passage channel C. Moreover, during exfoliation of the mucous membrane, no RF current flows through the electrosurgical knife 1.

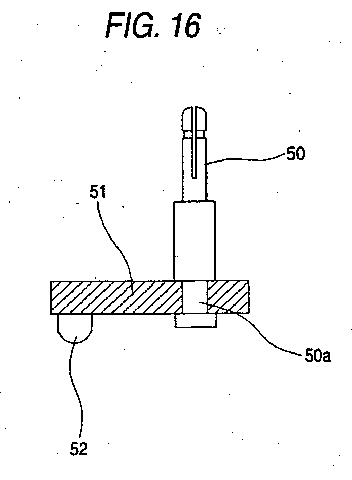

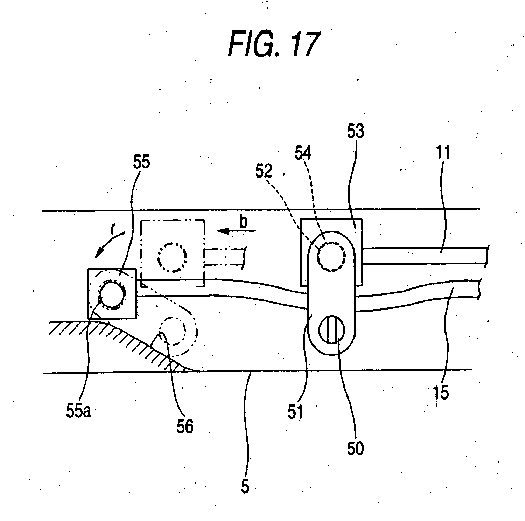

Here, in the second embodiment, by manually operating the slide electrode 45, the terminal pin 42 arranged on the slider was switched over to between the conductor line 11a of the flexible cord 11 leading to the electrosurgical knife 10 and to the wiring cable 15 leading to the RF current conductors 14. Alternatively, switchover is available in conjunction with the movement of the slider 41. As shown in Fig. 16, a swing arm 51 is coupled onto the terminal pin 50 arranged on the slider, to rotate about the axis 50a of the terminal pin 50. As shown in Fig. 17, the swing arm 51 is fit with a contact 52. The slider is fit with a clamp member 53 coupled to the flexible cord 11. The clamp member 53 has a contact 54 connected with the conductor wire 11a of the flexible cord 11. Meanwhile, a contact member 55 connected to the wiring cable 15 is fixed on the main stem 5 in a position closer to the base end. The contact member 55 is fit with a contact 55a.

Here, the main stem 5 is formed with a cam surface 56 in a position frontward of the arrangement of the contact member 55. When the slider is moved rearward, the cam surface 56 causes the swing arm 51 to rotate about the terminal pin 50 in conjunction with the operation thereof.

With this structure, when the slider is in a forward state, electric connection is available with the conductor wire 11a of the flexible cord 11 connected to the electrosurgical knife 10, as shown by the solid lines in Fig. 17. Meanwhile, when the slider is moved rearward along the arrow "b" in Fig. 17 into a position shown by the virtual line in the figure, the swing arm 51 having the contact 52 is guided on the cam surface 56 and rotated in a direction of the arrow "r" in conjunction with the operation thereof. As a result, the contact 52 is brought out of connection from the contact 54, thus automatically being changed into a state connected to the contact 55a that is connected to the wiring cable 15.

The two procedure sections, built in the flexible sheath at its tip, allow for two types of procedures, i.e. mucous-membrane incision and exfoliation. Those can perform the procedures continuously with smoothness and efficiency in the state being passed through the endoscopic treatment equipment-passage channel.

## Claims

1. An electrosurgical treatment equipment (1) comprising:
(i) an treatment equipment body comprising
a flexible sheath (2) that includes an electrical insulation material and is to be inserted in an treatment equipment-passage channel (C) of an endoscope (5),
an electrosurgical knife (10) arranged in the flexible sheath (2), and
a handle section (4) that is coupled to a base of the flexible sheath (2) and that advances and retracts the electrosurgical knife (10) at a tip of the flexible sheath (2); and
(ii) an RF power source (7) that applies a radio-frequency current to the electrosurgical knife (10) through a cord (11) coupled to the electrosurgical knife (10);
wherein the flexible sheath (2) comprises at least one RF current conductor (14) consisting of two electrode members in a manner being exposed in an outer peripheral surface at a tip of the flexible sheath (2), **characterised in that** the electrode members (14) are arranged in positions having an angular relationship of 180 degrees in an outer peripheral surface of the flexible sheath (2), the RF current conductor (14) having a predetermined length in an axial direction of the flexible sheath (2) and a predetermined width, wherein the predetermined width is smaller than the predetermined lenght, and the RF current conductor (14) having a straight elongated shape in the axial direction of the flexible sheath (2), the RF current conductor (14) being structured to be placed in and out of electrical connection with and from RF power source (7).

2. An electrosurgical treatment equipment (1) according to claim 1,
wherein the handle section (4) comprises:
a main stem (5) coupled to the base of the flexible sheath (2); and
a slider (6) slidably fit over the main stem (5) axially of the main stem (5) and coupled with another end of the cord (11),
wherein the electrosurgical knife (10) is restricted in a greatest protection out of the tip of the flexible sheath (2) by a projection restricting section (10b).

3. An electrosurgical treatment equipment (1) according to claim 1,
the both electrode members (14) are connected to a wiring (15a) received in the flexible sheath (2),
the wiring (15a) is connected to a contact (23) arranged on the main stem, and
the contact (23) is to be placed in and out of electrical connection with and from the RF power source (7) connected to the handle section (4).

4. An electrosurgical treatment equipment (1) according to claim 2,
wherein, when the slider (6) of the handle section (4) is slid over the main stem (5) to project the electrosurgical knife (10) out of the flexible sheath (2), electrical connection is provided between the RF power source (7) and the electrosurgical knife (10), and
when the electrosurgical knife (10) is retracted in the flexible sheath (2) by the slider (6), the RF current conductor (14), at a retraction stroke end, is electrically connected with the RF power source (7).

## Patentansprüche

1. Elektrochirurgische Behandlungsvorrichtung (1), umfassend:
(i) einen Behandlungsvorrichtungskörper, umfassend
eine flexible Hülle (2), die einen elektrischen Isolierstoff enthält und in einen Behandlungsvorrichtungs-Durchgangskanal (C) eines Endoskops (S) einzuführen ist,
ein elektrochirurgisches Messer (10), das in der flexiblen Hülle (2) angeordnet ist, und
einen Handhabungsteil (4), der mit einer Basis der flexiblen Hülle (2) gekoppelt ist, und der das elektrochirurgische Messer (10) an der Spitze der flexiblen Hülle (2) vorrückt und zurückzieht; und
(ii) eine HF-Energiequelle, die einen hochfrequenten Strom über ein mit dem elektrochirurgischen Messer (10) gekoppeltes Kabel an das elektrochirurgische Messer (10) liefert,
wobei die flexible Hülle (2) mindestens einen HF-Stromleiter (14), bestehend aus zwei Elektrodenelementen, in einer Weise aufweist, in der er in einer Außenumfangsfläche einer Spitze der flexiblen Hülle (2) freiliegt, **dadurch gekennzeichnet, dass** die Elektrodenelemente (14) an Stellen angeordnet sind, die eine Winkelbeziehung von 180° in einer Außenumfangsfläche der flexiblen Hülle (2) aufweisen, der HF-Stromleiter (14) eine vorbestimmte Länge in axialer Richtung der flexiblen Hülle (2) und eine vorbestimmte Breite, die kleiner ist als die vorbestimmte Länge, aufweist und der HF-Stromleiter (14) eine gestreckte lange Form in axialer Richtung der flexiblen Hülle (2) aufweist, der HF-Stromleiter (14) derart strukturiert ist, dass er in elektrischer Verbindung mit und aus elektrischer Verbindung von der HF-Energiequelle (7) plazierbar ist.

2. Elektrochirurgische Behandlungsvorrichtung (1) nach Anspruch 1, bei der der Handhabungsteil (4) aufweist:
einen Hauptstil (5), der mit der Basis der flexiblen Hülle (2) gekoppelt ist, und
ein Gleitstück (6), welches gleitfähig über den Hauptstil (5) in dessen axialer Richtung angesetzt ist und mit einem anderen Ende des Kabels (11) verbunden ist,
wobei das elektrochirurgische Messer (10) in seinem größtmöglichen Überstand über die Spitze der flexiblen Hülle (2) von einem Überstand-Einschränkungsabschnitt (10b beschränkt wird.

3. Elektrochirurgische Behandlungsvorrichtung (1) nach Anspruch 1, **dadurch gekennzeichnet, dass** die beiden Elektrodenelemente (14) mit einer in der flexiblen Hülle (2) aufgenommenen Verdrahtung (15a) verbunden sind,
die Verdrahtung (15a) mit einem an dem Hauptstil angeordneten Kontakt (23) verbunden ist, und
der Kontakt (23) in und aus elektrischer Verbindung mit bzw. von der HF-Energiequelle (7) bringbar ist, die an den Handhabungsteil (4) angeschlossen ist.

4. Elektrochirurgische Behandlungsvorrichtung (1) nach Anspruch 2, bei der, wenn das Gleitstück (6) des Handhabungsteils (4) über den Hauptstil (5) verschoben wird, um das elektrochirurgische Messer (10) aus der flexiblen Hülle (2) auszufahren, eine elektrische Verbindung zwischen der HF-Energiequelle (7) und dem elektrochirurgischen Messer (10) geschaffen wird, und
wenn das elektrochirurgische Messer (10) von dem Gleitstück (6) in die flexible Hülle (2) zurückgezogen wird, der HF-Stromleiter (14) an einem Rückhubende elektrisch mit der HF-Stromquelle (7) verbunden wird.

## Revendications

1. Equipement de traitement électro-chirurgical (1) comprenant :
(i) un corps d'équipement de traitement comprenant :
une gaine souple (2) qui comprend un matériau d'isolation électrique et qui doit être insérée dans un canal de passage d'équipement de traitement (C) d'un endoscope (5),
une lame électro-chirurgicale (10) agencée dans la gaine souple (2), et
une section de poignée (4) qui est couplée à une base de la gaine souple (2) et qui fait avancer et rétracte la lame électro-chirurgicale (10) au niveau d'une pointe de la gaine souple (2) ; et
(ii) une source de puissance RF (7) qui applique un courant de radiofréquence sur la lame électro-chirurgicale (10) par un cordon (11) couplé à la lame électro-chirurgicale (10),
dans lequel la gaine souple (2) comprend au moins un conducteur de courant RF (14) se composant de deux éléments d'électrode afin d'être exposé dans une surface périphérique externe au niveau d'une pointe de la gaine souple (2), **caractérisé en ce que** les éléments d'électrode sont agencés dans des positions ayant une relation angulaire de 180 degrés dans une surface périphérique de la gaine souple (2), le conducteur de courant RF (14) ayant une longueur prédéterminée dans une direction axiale de la gaine souple (2) et une largeur prédéterminée, dans lequel la largeur prédéterminée est inférieure à la longueur prédéterminée, et le conducteur de courant RF (14) ayant une forme droite allongée dans la direction axiale de la gaine souple (2), le conducteur de courant RF (14) étant structuré pour être placé en et hors de connexion électrique avec et de la source de puissance RF (7).

2. Equipement de traitement électro-chirurgical (1) selon la revendication 1,
dans lequel la section de poignée (4) comprend :
une tige principale (5) couplée à la base de la gaine souple (2) ;
une glissière (6) montée de manière coulissante sur la tige principale (5) axialement par rapport à la tige principale (5) et couplée avec une autre extrémité du cordon (11),
dans lequel la lame électro-chirurgicale (10) est retenue dans la plus grande saillie hors de la pointe de la gaine souple (2) par une section de retenue de saillie (10b).

3. Equipement de traitement électro-chirurgical (1) selon la revendication 1,
**Caractérisé en ce que** les deux éléments d'électrode (14) sont raccordés à un câblage (15a) reçu dans la gaine souple (2),
le câblage (15a) est raccordé à un contact (23) agencé sur la tige principale, et
le contact (23) doit être placé en et hors de connexion électrique avec et de la source de puissance RF (7) raccordée à la section de poignée (4).

4. Equipement de traitement électro-chirurgical (1) selon la revendication 2,
dans lequel, lorsque la glissière (6) de la section de poignée (4) coulisse sur la tige principale (5) pour faire sortie la lame électro-chirurgicale (10) de la gaine souple (2), la connexion électrique est fournie entre la source de puissance RF (7) et la lame électro-chirurgicale (10), et
lorsque la lame électro-chirurgicale (10) est rétractée dans la gaine souple (2) par la glissière (6), le conducteur de courant RF (14), au niveau d'une fin de course de rétraction, est électriquement raccordé avec la source de puissance RF (7).
